# EUROPEAN PATENT APPLICATION

(11) **EP 4 570 806 A1**
(43) Date of publication of application: **18.06.2025**
(21) Application number: 23851631.4
(22) Date of filing: 31.07.2023
(51) Int. Cl.: C07D 513/22, C07D 403/12, C07D 401/14, A61K 39/395, A61K 31/495, A61P 35/00

(54) **COMPOSITION OF PIPERAZINE COMPOUND AND PD-1 INHIBITOR OR PD-L1 INHIBITOR AND USE THEREOF IN TREATING TUMORS**

(30) Priority: 09.08.2022 CN 202210949146
(71) Applicant: Kangbaida (Sichuan) Biotechnology Co., Ltd., Chengdu, Sichuan 610000 (CN)
(72) Inventor: ZHU, Yuqin, Chengdu, Sichuan 610000 (CN); WEI, Yonggang, Chengdu, Sichuan 610000 (CN); CHU, Hongzhu, Chengdu, Sichuan 610000 (CN); YE, Fei, Chengdu, Sichuan 610000 (CN); SU, Guizhuan, Chengdu, Sichuan 610000 (CN); SUN, Yi, Chengdu, Sichuan 610000 (CN)
(74) Representative: Kador & Partner Part mbB
(86) International application number: PCT/CN2023/110231
(87) International publication number: WO 2024/032410

(57) **Abstract**

The present application relates to a combination of a piperazine compound and a PD-1 inhibitor antibody or a PD-L1 inhibitor antibody and use thereof in treating tumors.

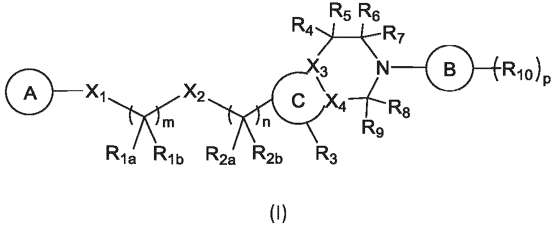

## Description

### Technical Field

The present application belongs to the technical field of medicines, and relates to a pharmaceutical combination which may exert antitumor effects. Specifically, the present application relates to a composition based on a piperazine compound and a PD-1 inhibitor antibody or a PD-L1 inhibitor and the use thereof in treating tumors.

### Background Art

Programmed cell death protein 1 (PD-1, Pdcd-1 or CD279) is a 55KD receptor protein associated with the CD28/CTLA4 co-stimulatory/inhibitory receptor family. Cancer cells express the ligand of PD-1, namely PD-L1, which allows them to evade the host immune system. The PD-1 inhibitors available on the market can significantly prolong the overall survival of patients by interfering with the immunosuppressive mechanism of tumors. However, this treatment method can only elicit a response in some patients, or some patients may respond well to the first dose, but the efficacy decreases or disappears after a period of administration. Therefore, there is an urgent need for new and improved therapies to enhance the therapeutic efficacy of existing therapies.

### Summary

The PCT application PCT/CN2022/094124 by the present applicant discloses the use of a piperazine derivative in treating cancers, in which the compounds described in the specification have high selectivity and significant inhibitory activity against PARP7. In subsequent studies, it was unexpectedly found that the piperazine derivative in combination with a PD-1 inhibitor antibody can significantly increase antitumor effects of the PD-1 inhibitor antibody or PD-L1 inhibitor antibody.

Therefore, the present disclosure aims to provide a composition of a piperazine compound and a PD-1 inhibitor antibody or a PD-L1 inhibitor antibody and use thereof in treating tumors.

One or more embodiments of the present disclosure provide a pharmaceutical combination of at least two components comprising component A being a PD-1 inhibitor antibody or a PD-L1 inhibitor antibody, and component B being a compound of formula (I), or a pharmaceutically acceptable salt, a stereoisomer, or a deuterated compound thereof: 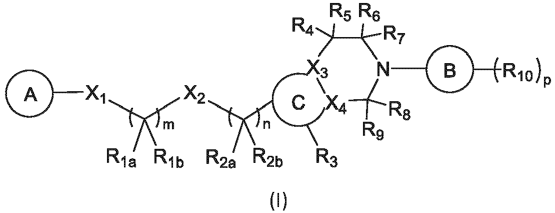 wherein
X₁ is NH, O or 4- to 6-membered heterocycle containing 1 to 3 heteroatoms selected from N and O;
X₂ is O or a single bond;
X₃ and X₄ are each independently C or N;
R₁ₐ and R_{1b} are each independently H, D or C₁₋₆ alkyl; or R₁ₐ and R_{1b} together with the carbon atom connected thereto form 3- to 5-membered cycloalkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl; or R₆ and R₇ together with the carbon atom connected thereto form C=O;
R₈ and R₉ are each independently H, D or C₁₋₆ alkyl; or R₈ and R₉ together with the carbon atom connected thereto form C=O; or R₈ and R₉ together with the carbon atom connected thereto form 3- to 5-membered cycloalkyl;
each R₁₀ is independently C₁₋₆ alkyl, C₁₋₆ alkoxy, CONR₁₀ₐR_{10b}, halogen, cyano, S(O)₂R_{10c}, SR_{10d} or 3-to 5-membered cycloalkyl, wherein the C₁₋₆ alkyl and C₁₋₆alkoxy are optionally substituted with 1 to 3 halogens;
R₁₀ₐ, R_{10b}, R_{10c} and R_{10d} are each independently H, D or C₁₋₆ alkyl;
A is 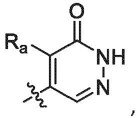 and Rₐ is C₁₋₆ alkyl, C₃₋₅ cycloalkyl, halogen or cyano, wherein the C₁₋₆ alkyl is optionally substituted with 1 to 3 halogens;
B is 5- to 10-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

One or more embodiments of the present disclosure provide a pharmaceutical combination of at least two components comprising component A being a PD-1 inhibitor antibody, and component B being a compound of formula (I), or a pharmaceutically acceptable salt, a stereoisomer, or a deuterated compound thereof, wherein
X₁ is NH, O or 4- to 6-membered heterocycle containing 1 to 3 heteroatoms selected from N and O;
X₂ is O or a single bond;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
each R₁₀ is independently C₁₋₆ alkyl, C₁₋₆ alkoxy, CONR₁₀ₐR_{10b}, halogen, cyano, S(O)₂R_{10c}, SR_{10d} or 3-to 5-membered cycloalkyl, wherein the C₁₋₆ alkyl and C₁₋₆alkoxy are optionally substituted with 1 to 3 halogens;
R₁₀ₐ, R_{10b}, R_{10c} and R_{10d} are each independently H, D or C₁₋₆ alkyl;
A is 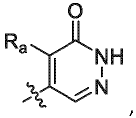 and Rₐ is C₁₋₆ alkyl, C₃₋₅ cycloalkyl, halogen or cyano, wherein the C₁₋₆ alkyl is optionally substituted with 1 to 3 halogens;
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3;
n is 0, 1, 2 or 3; and
p is 0, 1, 2 or 3.

One or more embodiments of the present disclosure provide a pharmaceutical combination of at least two components comprising component A being a PD-1 inhibitor antibody, and component B being a compound of formula (I-1), or a pharmaceutically acceptable salt, a stereoisomer, or a deuterated compound thereof: 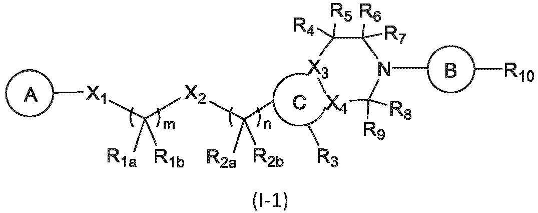 wherein
X₁ is NH or 4- to 6-membered heterocycle containing 1 to 3 heteroatoms selected from N and O;
X₂ is O;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 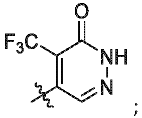
B is 5- to 6-membered carbocycle or heterocycle, wherein the heterocycle contains 1 to 3 heteroatoms selected from N, O and S;
C is 5- to 6-membered heterocycle containing 1 to 3 heteroatoms of N;
m is 1, 2 or 3; and
n is 0, 1, 2 or 3.

One or more embodiments of the present disclosure provide a pharmaceutical combination of at least two components comprising component A being a PD-1 inhibitor antibody, and component B being a compound of formula (I-2), or a pharmaceutically acceptable salt, a stereoisomer, or a deuterated compound thereof: 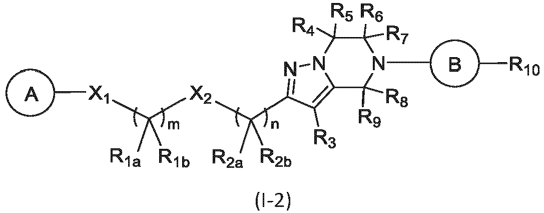 wherein
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 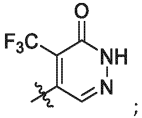
B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present disclosure provide a pharmaceutical combination of at least two components comprising component A being a PD-1 inhibitor antibody, and component B being a compound of formula (I-2), or a pharmaceutically acceptable salt, a stereoisomer, or a deuterated compound thereof, wherein
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 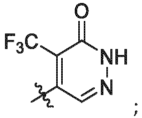
B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present disclosure provide a pharmaceutical combination of at least two components comprising component A being a PD-1 inhibitor antibody, and component B being a compound of formula (I-2), or a pharmaceutically acceptable salt, a stereoisomer, or a deuterated compound thereof, wherein
X₁ is selected from NH;
X₂ is selected from O;
R₁ₐ and R_{1b} are each independently selected from H, D or C₁₋₆ alkyl;
R₂ₐ and R_{2b} are each independently selected from H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D or C₁₋₆ alkyl;
A is 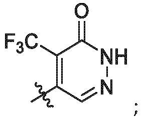
B is 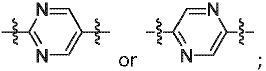
m is 1, 2 or 3; and
n is 0, 1, 2 or 3.

One or more embodiments of the present disclosure provide a pharmaceutical combination of at least two components comprising component A being a PD-1 inhibitor antibody, and component B being a compound of formula (I-2), or a pharmaceutically acceptable salt, a stereoisomer, or a deuterated compound thereof, wherein
X₁ is selected from NH;
X₂ is selected from O;
R₁ₐ and R_{1b} are each independently selected from H, D or C₁₋₃ alkyl;
R₂ₐ and R_{2b} are each independently selected from H, D or C₁₋₃ alkyl;
R₃ is selected from H, D or CF₃;
R₄ and R₅ are each independently selected from H or D;
R₆ and R₇ are each independently selected from H or D;
R₈ and R₉ are each independently selected from H or D;
R₁₀ is CF₃;
A is 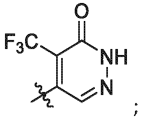
B is 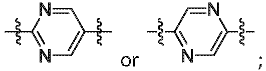
m is 1, 2 or 3; and
n is 0, 1 or 2.

One or more embodiments of the present disclosure provide a pharmaceutical combination of at least two components comprising component A being a PD-1 inhibitor antibody, and component B being a compound of formula (I-3), or a pharmaceutically acceptable salt, a stereoisomer, or a deuterated compound thereof: 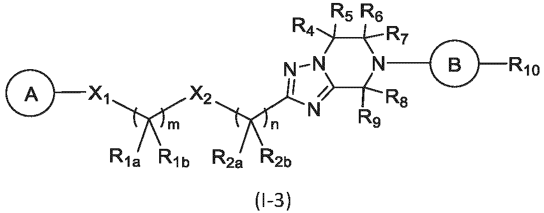 wherein
X₁ is NH or 4- to 6-membered heterocycle containing 1 to 3 heteroatoms selected from N and O;
X₂ is O;
R₁ₐ and R_{1b} are each independently H, D or C₁₋₆ alkyl;
R₂ₐ and R_{2b} are each independently H, D or C₁₋₆ alkyl;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 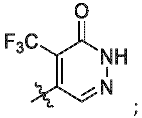
B is 5- to 6-membered carbocycle or heterocycle, wherein the heterocycle contains 1 to 3 heteroatoms selected from N, O and S;
m is 1, 2 or 3; and
n is 0, 1, 2 or 3.

One or more embodiments of the present disclosure provide a pharmaceutical combination of at least two components comprising component A being a PD-1 inhibitor antibody, and component B being a compound of formula (I-3), or a pharmaceutically acceptable salt, a stereoisomer, or a deuterated compound thereof, wherein
X₁ is NH or 4- to 6-membered heterocycle containing 1 to 3 heteroatoms selected from N and O;
X₂ is O;
R₁ₐ and R_{1b} are each independently H, D or C₁₋₃ alkyl;
R₂ₐ and R_{2b} are each independently H, D or C₁₋₃ alkyl;
R₄ and R₅ are each independently H, D or C₁₋₃ alkyl;
R₆ and R₇ are each independently H, D or C₁₋₃ alkyl;
R₈ and R₉ are each independently H, D or C₁₋₃ alkyl; or R₈ and R₉ together with the carbon atom connected thereto form C=O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 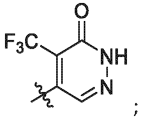
B is 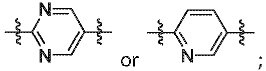
m is 1, 2 or 3; and
n is 0, 1 or 2.

One or more embodiments of the present disclosure provide a pharmaceutical combination of at least two components comprising component A being a PD-1 inhibitor antibody, and component B being a compound of formula (I-3), or a pharmaceutically acceptable salt, a stereoisomer, or a deuterated compound thereof, wherein
X₁ is NH;
X₂ is O;
R₁ₐ and R_{1b} are each independently H, D or C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 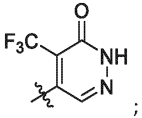
B is 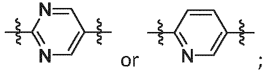
m is 1, 2 or 3; and
n is 0, 1 or 2.

One or more embodiments of the present disclosure provide a pharmaceutical combination of at least two components comprising component A being a PD-1 inhibitor antibody, and component B selected from compounds as shown below, or a pharmaceutically acceptable salt, a stereoisomer, or a deuterated compound thereof: 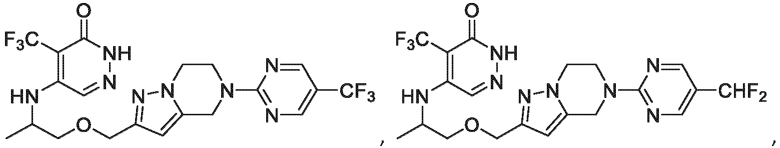 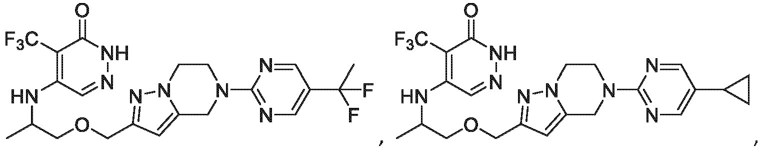 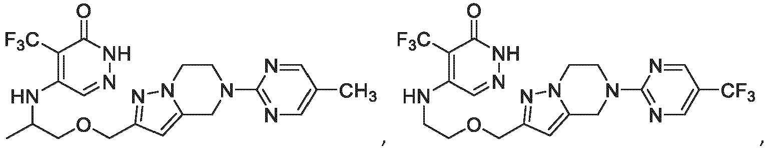 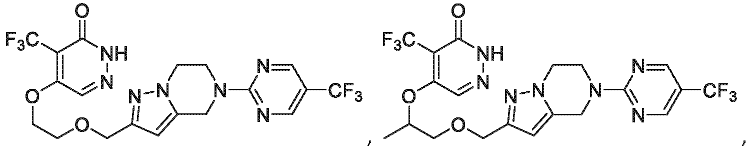 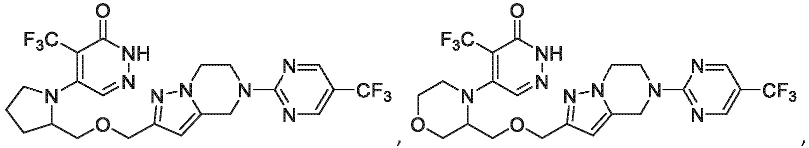 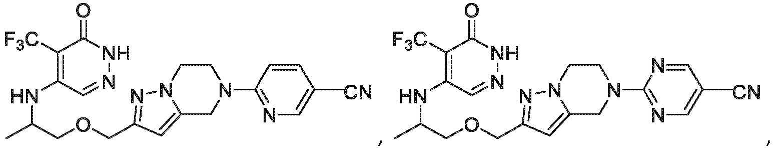 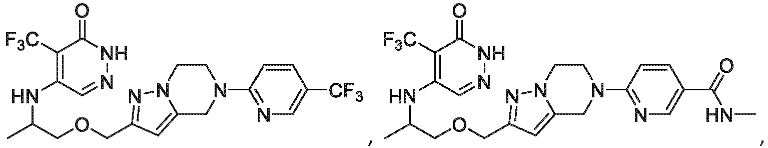 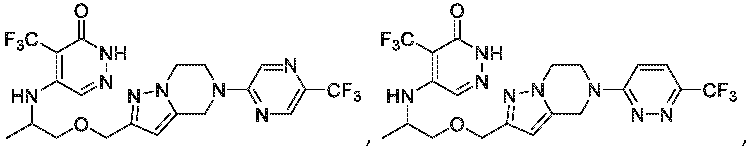 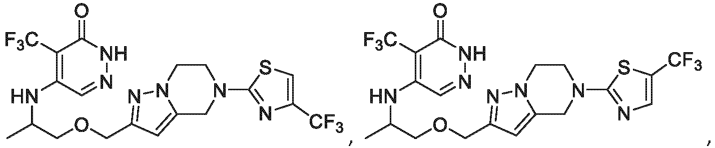 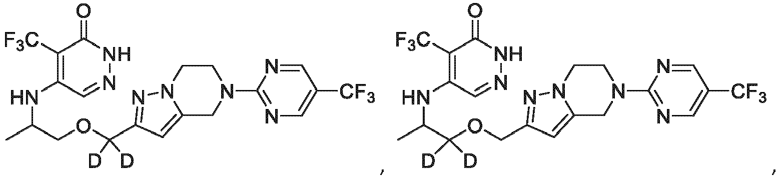 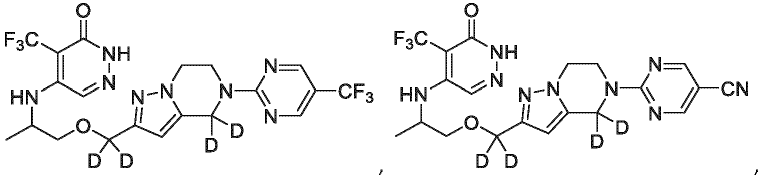 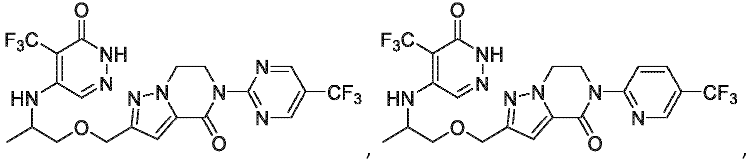 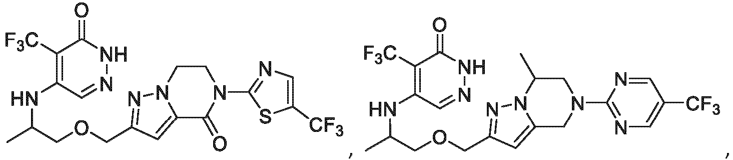 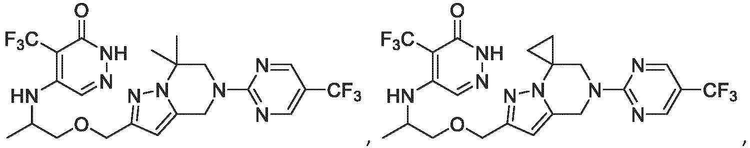 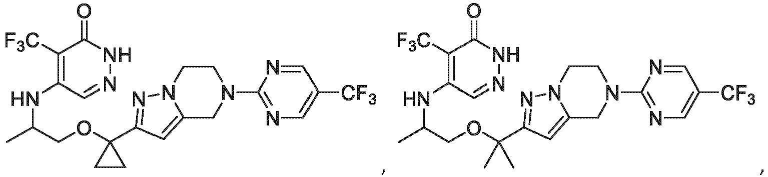 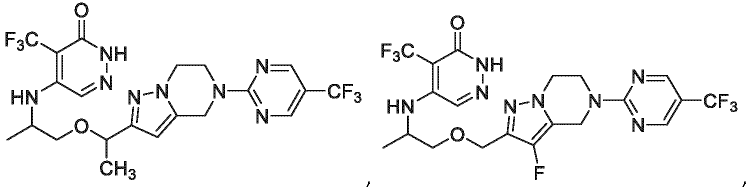 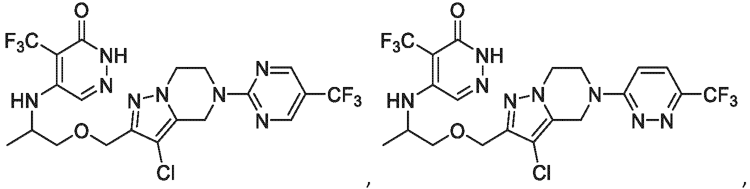 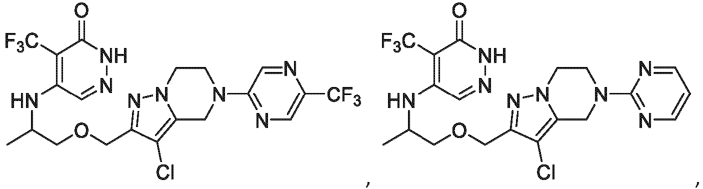 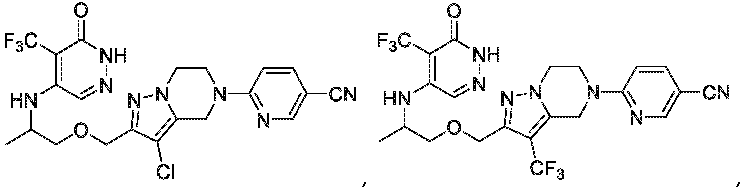 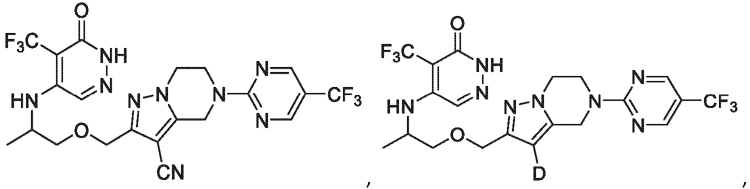 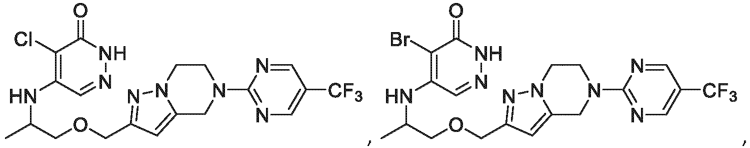 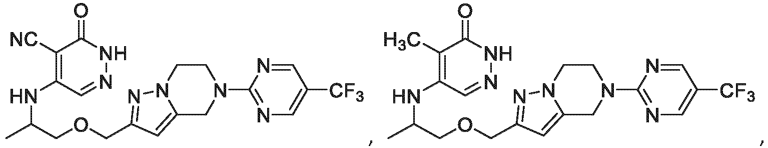 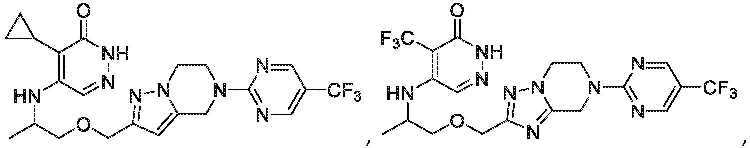 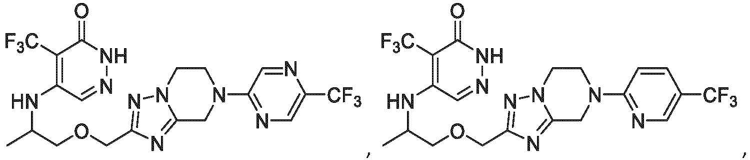 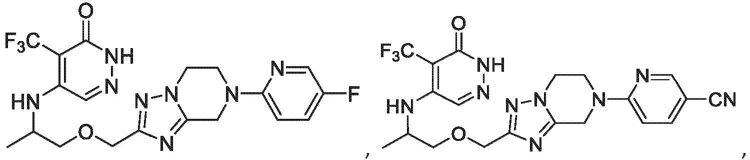 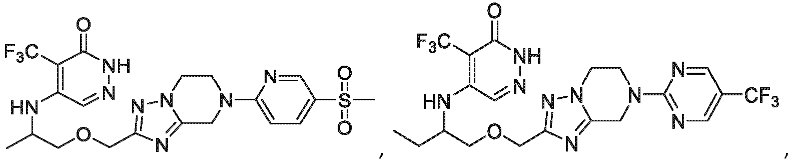 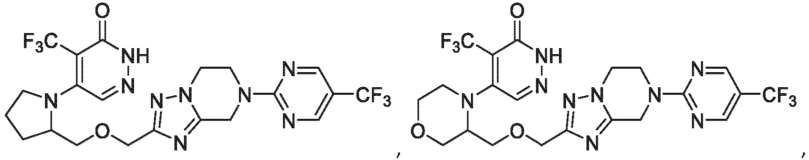 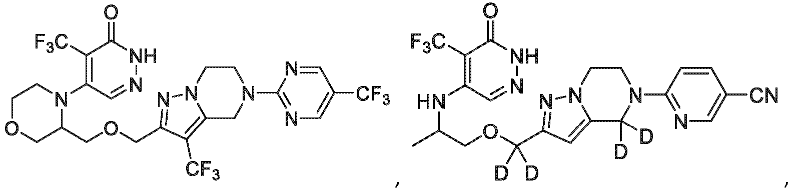 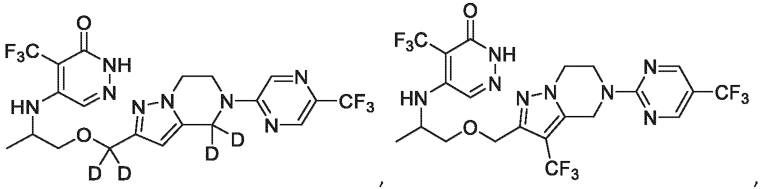 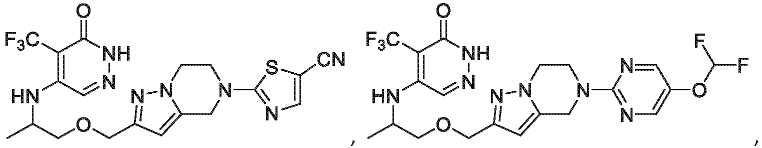 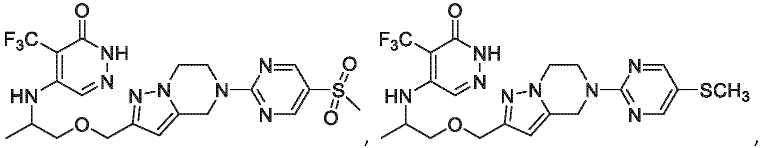 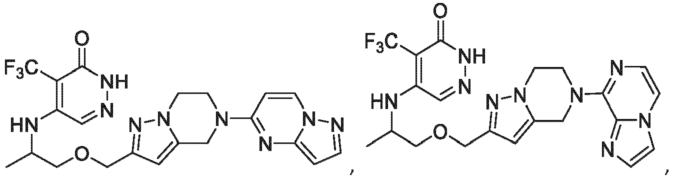 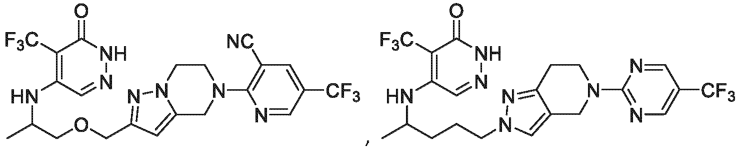 or

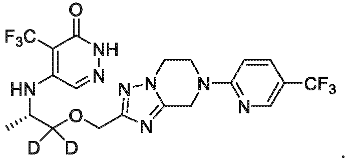

One or more embodiments of the present disclosure provide a pharmaceutical combination of at least two components, wherein the component A is Pembrolizumab, Nivolumab, Serplulimab, Pidilizumab, Lambrolizumab, Atezolizumab, Toripalimab, Sintilimab, Tislelizumab, Camrelizumab, Penpulimab, Zimberelimab, Envafolimab, Sugemalimab, Dostarlimab, Cadonilimab, Cemiplimab, Retifanlimab, BMS-986213, HX-008, Geptanolimab, Prolgolimab, Socazolimab, Avelumab, Adebrelimab, or Durvalumab.

In one or more embodiments of the present disclosure, the pharmaceutical combination comprises the above-described component A and the above-described component B, and one or more pharmaceutically acceptable excipients, diluents or carriers.

In one or more embodiments of the present disclosure, the total daily dosage of the component B is selected from 50-1500 mg, preferably 100-1000 mg, more preferably 400-800 mg, as measured by the amount of free base.

In one or more embodiments of the present disclosure, the present disclosure relates to the use of the pharmaceutical combination in treating or preventing solid tumors.

In one or more embodiments of the present disclosure, the solid tumor is selected from non-small cell lung cancer, head and neck squamous carcinoma, esophageal squamous carcinoma, hormone-receptor-positive (HR+) breast cancer, advanced solid tumor with PARP7 expansion, colon cancer or lung cancer, preferably colon cancer or lung cancer, more preferably colon cancer.

In one or more embodiments of the present disclosure, the above-described component A and the above-described component B are given simultaneously.

In one or more embodiments of the present disclosure, the above-described component A and the above-described component B are given sequentially. For example, the component A is given first, then the component B is given, or the component B is given first, then the component A is given. The administration intervals may be in hours, days, weeks, or months.

In one or more embodiments of the present disclosure, the above-described component A is given intravenously at a frequency of twice a week, once a week, once every two weeks, once every three weeks, once every four weeks, once every month, once every 3-6 months, preferably twice a week, once a week, once every two weeks or once every three weeks.

In one or more embodiments of the present disclosure, the above-described component B is given orally at a frequency of thrice daily, twice daily, once daily, once every two days, once every three days, once every four days, once every five days, once every six days, once a week, once every two weeks, once every three weeks, or once every four weeks, preferably twice daily or once daily.

### Advantages of the present disclosure

The combination of compound of the present disclosure and a PD-1 inhibitor or a PD-L1 inhibitor, has a better *in vivo* antitumor effect than the PD-1 inhibitor or the PD-L1 inhibitor monotherapy and exhibits good safety and tolerance. It is shown that the pharmaceutical combination of the present disclosure has a significant synergistic effect.

### Brief Description of the Drawings

FIG. 1 shows the structural formula of the compound ①.
FIG. 2 shows growth curves of tumor volume of tumor-bearing mice.
FIG. 3 shows percent body weight change of tumor-bearing mice.

### Detailed Description of Embodiments

The implementation process and beneficial effects of the present disclosure are described in detail below by way of specific examples, which are intended to help readers better understand the essence and characteristics of the present disclosure but not to limit the scope of implementation of the present disclosure.

The present disclosure will be described in further detail below with reference to the accompanying drawings.

The compound ① in the examples is compound 1 of PCT application PCT/CN2022/094124 and is prepared according to the preparation method therefor.

### Efficacy test in CT26 mouse tumor-bearing model

### 1. Experimental steps

### 1.1 Cell culture

Colon cancer cells CT26 were purchased from ATCC for culture under conditions of a DMEM medium supplemented with 10% fetal bovine serum and 1% penicillin and streptomycin, in a cell incubator containing 5% CO₂ at 37°C. The cells in an exponential growth phase were digested with trypsin, and the cells were collected and counted, followed by inoculation.

### 1.2. Grouping and administration

BALB/c mice were adapted to the laboratory environment and then inoculated subcutaneously with a CT26 cell suspension in the right flank at an inoculation amount of 1 × 10⁶ cells/mouse, with an inoculation volume of 0.1 mL. When the tumors were grown to approximately 30-80 mm³, 32 animals were screened for grouping. The animals were randomly divided into 4 groups based on the tumor volume, with 8 mice in each group. The day of grouping is defined as day 0 (PG-D0) of the experiment, and administration began on day 1. The grouping and administration regimen are shown in Table 1.

**Table 1. Administration regimen**

| Group No. | Group | Mode of administration | Number of animals |
|---|---|---|---|
| 1 | Blank group | - | 8 |
| 2 | Compound ① | Compound ① 125 mg/kg, BID, i.g | 8 |
| 3 | anti-PD-1 | anti-PD-1 5 mg/kg, BIW, i.p | 8 |
| 4 | Compound ① + anti-PD-1 | Compound ① 125 mg/kg, BID, i.g + anti-PD-1 5 mg/kg, BIW, i.p | 8 |

| | | | |
|---|---|---|---|
| Notes: i.g. refers to intragastrical gavage; i.p. refers to intraperitoneal administration; BID: dosing twice daily; and BIW refers to dosing twice a week. | | | |

### 2. Assay method

### 2.1 Tumor volume

The diameter of tumors was measured twice a week using a vernier caliper to calculate the volume of tumors, and a tumor growth curve was plotted. The calculation formula of the tumor volume (V) is:
V = 1/2 × a × b², where a and b represent the long and short diameters of the tumor, respectively.

### 2.2 Body weight of mice

Mice were weighed at least twice a week during a drug treatment cycle.

### 3. Experimental results

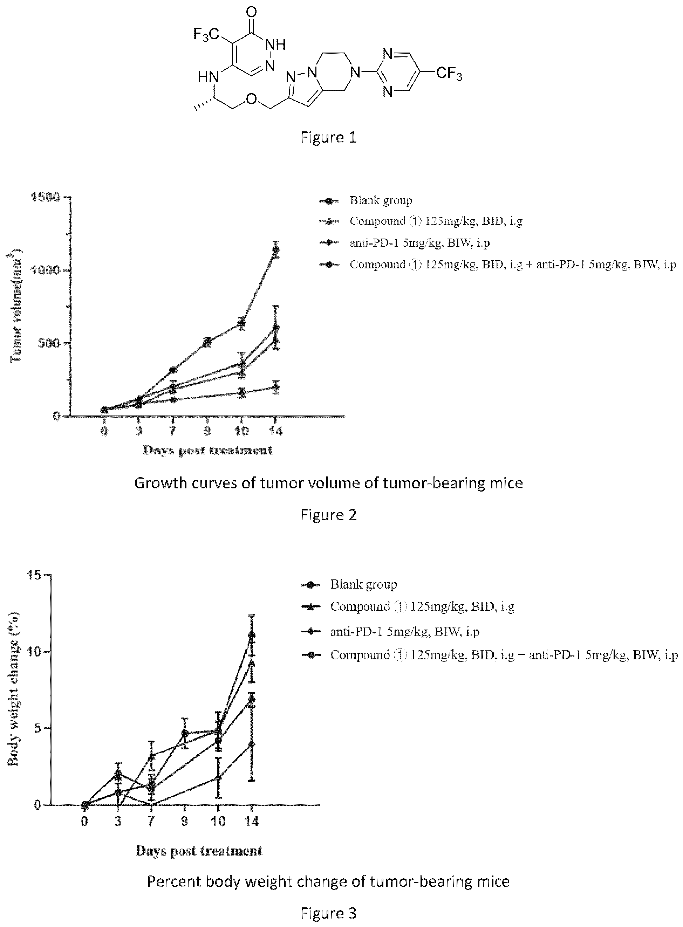

As shown in FIG. 2 and FIG. 3, the combination of compound ① and an anti-PD-1 antibody has a better *in vivo* antitumor effect than the anti-PD-1 antibody alone and shows good safety and tolerance. It is shown that the pharmaceutical combination of the present disclosure has a significant synergistic effect.

Specific embodiments have been described in detail in the specification of the present disclosure. A person skilled in the art should recognize that the embodiments described above are exemplary and cannot be construed as limiting the present disclosure. Additionally, a person skilled in the art can make several improvements and modifications to the present disclosure without departing from the principle of the present disclosure, and the technical solutions obtained based on these improvements and modifications also fall within the scope of protection of the claims of the present disclosure.

## Claims

1. A pharmaceutical combination of at least two components comprising component A being a PD-1 inhibitor antibody or a PD-L1 inhibitor antibody, and component B being a compound of formula (I), or a pharmaceutically acceptable salt, a stereoisomer, or a deuterated compound thereof: 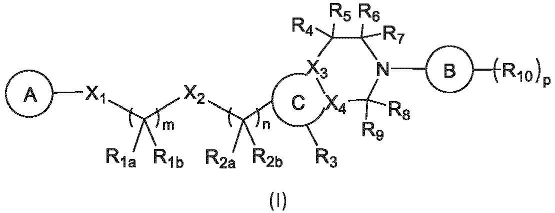 wherein
X₁ is NH, O or 4- to 6-membered heterocycle containing 1 to 3 heteroatoms selected from N and O;
X₂ is O or a single bond;
X₃ and X₄ are each independently C or N;
R₁ₐ and R_{1b} are each independently H, D or C₁₋₆ alkyl; or R₁ₐ and R_{1b} together with the carbon atom connected thereto form 3- to 5-membered cycloalkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl; or R₆ and R₇ together with the carbon atom connected thereto form C=O;
R₈ and R₉ are each independently H, D or C₁₋₆ alkyl; or R₈ and R₉ together with the carbon atom connected thereto form C=O; or R₈ and R₉ together with the carbon atom connected thereto form 3- to 5-membered cycloalkyl;
each R₁₀ is independently C₁₋₆ alkyl, C₁₋₆ alkoxy, CONR₁₀ₐR_{10b}, halogen, cyano, S(O)₂R_{10c}, SR_{10d} or 3-to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 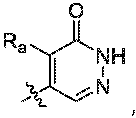 and Rₐ is C₁₋₆ alkyl, C₃₋₅ cycloalkyl, halogen or cyano, wherein the C₁₋₆ alkyl is optionally substituted with 1 to 3 halogens;
B is 5- to 10-membered carbocycle or heterocycle, wherein the heterocycle contains 1 to 3 heteroatoms selected from N, O and S;
Cis 5- to 6-membered heterocycle containing 1 to 3 heteroatoms of N;
m is 1, 2 or 3;
n is 0, 1, 2 or 3; and
p is 0, 1, 2 or 3.

2. The pharmaceutical combination according to claim 1, wherein the component B is a compound of formula (I), or a pharmaceutically acceptable salt, a stereoisomer, or a deuterated compound thereof, wherein
X₁ is NH, O or 4- to 6-membered heterocycle containing 1 to 3 heteroatoms selected from N and O;
X₂ is O or a single bond;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl; or R₂ₐ and R_{2b} together with the carbon atom connected thereto form 3- to 5-membered cycloalkyl;
R₃ is H, D, C₁₋₆ alkyl, halogen or cyano, wherein the C₁₋₆ alkyl is optionally substituted with 1 to 3 halogens;
R₄ and R₅ are each independently H, D or C₁₋₆ alkyl; or R₄ and R₅ together with the carbon atom connected thereto form 3- to 5-membered cycloalkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
each R₁₀ is independently C₁₋₆ alkyl, C₁₋₆ alkoxy, CONR₁₀ₐR_{10b}, halogen, cyano, S(O)₂R_{10c}, SR_{10d} or 3-to 5-membered cycloalkyl, wherein the C₁₋₆ alkyl and C₁₋₆ alkoxy are optionally substituted with 1 to 3 halogens;
R₁₀ₐ, R_{10b}, R_{10c} and R_{10d} are each independently H, D or C₁₋₆ alkyl;
A is 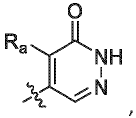 and Rₐ is C₁₋₆ alkyl, C₃₋₅ cycloalkyl, halogen or cyano, wherein the C₁₋₆ alkyl is optionally substituted with 1 to 3 halogens;
B is 5- to 6-membered carbocycle or heterocycle, wherein the heterocycle contains 1 to 3 heteroatoms selected from N, O and S;
Cis 5- to 6-membered heterocycle containing 1 to 3 heteroatoms of N;
m is 1, 2 or 3;
n is 0, 1, 2 or 3; and
p is 0, 1, 2 or 3.

3. The pharmaceutical combination according to claim 2, wherein the component B is a compound of formula (I-1), or a pharmaceutically acceptable salt, a stereoisomer, or a deuterated compound thereof, wherein 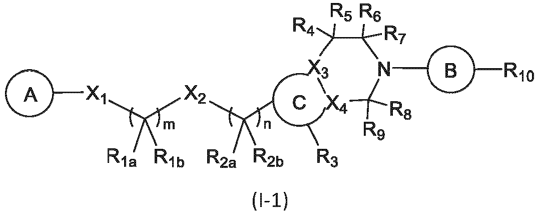 wherein
X₁ is NH or 4- to 6-membered heterocycle containing 1 to 3 heteroatoms selected from N and O; X₂ is O;
X₃ and X₄ are each independently C or N;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 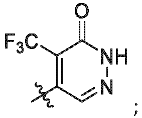
B is 5- to 6-membered carbocycle or heterocycle, wherein the heterocycle contains 1 to 3 heteroatoms selected from N, O and S;
Cis 5- to 6-membered heterocycle containing 1 to 3 heteroatoms of N;
m is 1, 2 or 3; and
n is 0, 1, 2 or 3.

4. The pharmaceutical combination according to claim 3, wherein the component B is a compound of formula (I-2), or a pharmaceutically acceptable salt, a stereoisomer, or a deuterated compound thereof, wherein 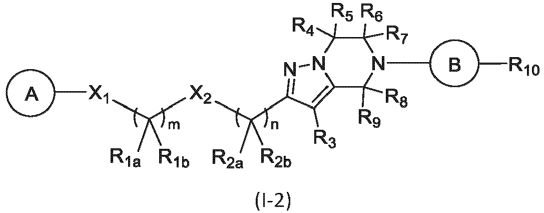 wherein
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 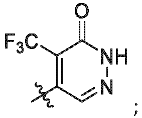
B is 5- to 6-membered carbocycle or heterocycle, wherein the heterocycle contains 1 to 3 heteroatoms selected from N, O and S;
m is 1, 2 or 3; and
n is 0, 1, 2 or 3.

5. The pharmaceutical combination according to claim 4, wherein the component B is a compound of formula (I-2), or a pharmaceutically acceptable salt, a stereoisomer, or a deuterated compound thereof, wherein
X₁ is NH;
X₂ is O;
R₁ₐ and R_{1b} are each independently H, D or C₁₋₆ alkyl;
R₂ₐ and R_{2b} are each independently H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 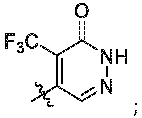
B is 5- to 6-membered carbocycle or heterocycle, wherein the heterocycle contains 1 to 3 heteroatoms selected from N, O and S;
m is 1, 2 or 3; and
n is 0, 1, 2 or 3.

6. The pharmaceutical combination according to claim 5, wherein the component B is a compound of formula (I-2), or a pharmaceutically acceptable salt, a stereoisomer, or a deuterated compound thereof, wherein
X₁ is selected from NH;
X₂ is selected from O;
R₁ₐ and R_{1b} are each independently selected from H, D or C₁₋₆ alkyl;
R₂ₐ and R_{2b} are each independently selected from H, D or C₁₋₆ alkyl;
R₃ is H, D, C₁₋₆ alkyl or halogen, wherein the C₁₋₆ alkyl is optionally substituted with 1 to 3 halogens;
R₄ and R₅ are each independently H or D;
R₆ and R₇ are each independently H or D;
R₈ and R₉ are each independently H or D;
R₁₀ is CF₃ or SR_{10d};
R_{10d} is H, D or C₁₋₆ alkyl;
A is 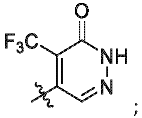
B is 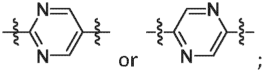
m is 1, 2 or 3; and
n is 0, 1, 2 or 3.

7. The pharmaceutical combination according to claim 6, wherein the component B is a compound of formula (I-2), or a pharmaceutically acceptable salt, a stereoisomer, or a deuterated compound thereof, wherein
X₁ is selected from NH;
X₂ is selected from O;
R₁ₐ and R_{1b} are each independently selected from H, D or C₁₋₃ alkyl;
R₂ₐ and R_{2b} are each independently selected from H, D or C₁₋₃ alkyl;
R₃ is selected from H, D or CF₃;
R₄ and R₅ are each independently selected from H or D;
R₆ and R₇ are each independently selected from H or D;
R₈ and R₉ are each independently selected from H or D;
R₁₀ is CF₃;
A is 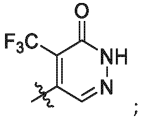
B is 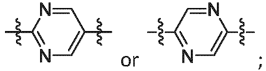
m is 1, 2 or 3; and
n is 0, 1 or 2.

8. The pharmaceutical combination according to claim 1, wherein the component B is a compound of formula (I-3), or a pharmaceutically acceptable salt, a stereoisomer, or a deuterated compound thereof, wherein 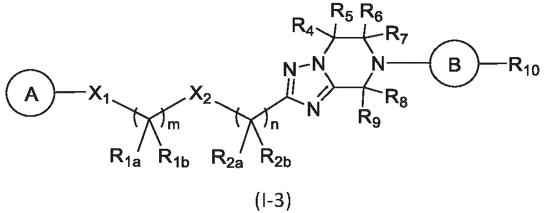 wherein
X₁ is NH or 4- to 6-membered heterocycle containing 1 to 3 heteroatoms selected from N and O; X₂ is O;
R₁ₐ and R_{1b} are each independently H, D or C₁₋₆ alkyl;
R₂ₐ and R_{2b} are each independently H, D or C₁₋₆ alkyl;
R₄ and R₅ are each independently H, D or C₁₋₆ alkyl;
R₆ and R₇ are each independently H, D or C₁₋₆ alkyl;
R₈ and R₉ are each independently H, D or C₁₋₆ alkyl; or R₈ and R₉ together with the carbon atom connected thereto form C=O;
R₁₀ is C₁₋₆ alkyl, C₁₋₆ alkoxy, cyano or SR_{10d}, wherein the C₁₋₆ alkyl and the C₁₋₆ alkoxy are optionally substituted with 1 to 3 halogens;
R_{10d} is H, D or C₁₋₆ alkyl;
A is 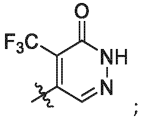
B is 5- to 6-membered carbocycle or heterocycle, wherein the heterocycle contains 1 to 3 heteroatoms selected from N, O and S;
m is 1, 2 or 3; and
n is 0, 1, 2 or 3.

9. The pharmaceutical combination according to claim 8, wherein the component B is a compound of formula (I-3), or a pharmaceutically acceptable salt, a stereoisomer, or a deuterated compound thereof, wherein
X₁ is NH or 4- to 6-membered heterocycle containing 1 to 3 heteroatoms selected from N and O; X₂ is O;
R₁ₐ and R_{1b} are each independently H, D or C₁₋₃ alkyl;
R₂ₐ and R_{2b} are each independently H, D or C₁₋₃ alkyl;
R₄ and R₅ are each independently H, D or C₁₋₃ alkyl;
R₆ and R₇ are each independently H, D or C₁₋₃ alkyl;
R₈ and R₉ are each independently H, D or C₁₋₃ alkyl; or R₈ and R₉ together with the carbon atom connected thereto form C=O;
R₁₀ is C₁₋₆ alkyl, cyano or SR_{10d}, wherein the C₁₋₆ alkyl is optionally substituted with 1 to 3 halogens;
R_{10d} is H, D or C₁₋₆ alkyl;
A is 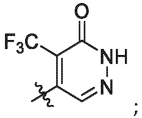
B is 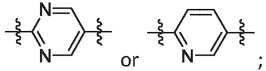
m is 1, 2 or 3; and
n is 0, 1 or 2.

10. The pharmaceutical combination according to claim 9, wherein the component B is a compound of formula (I-3), or a pharmaceutically acceptable salt, a stereoisomer, or a deuterated compound thereof, wherein
X₁ is NH;
X₂ is O;
R₁ₐ and R_{1b} are each independently H, D or C₁₋₃ alkyl;
R₂ₐ and R_{2b} are each independently H or D;
R₄ and R₅ are each independently H or D;
R₆ and R₇ are each independently H or D;
R₈ and R₉ are each independently H or D;
R₁₀ is CF₃;
A is 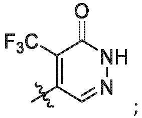
B is 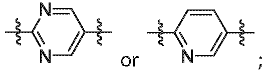
m is 1, 2 or 3; and
n is 0, 1 or 2.

11. The pharmaceutical combination according to any one of claims 1 to 10, wherein the component B is selected from compounds as shown below, or a pharmaceutically acceptable salt, a stereoisomer, or a deuterated compound thereof: 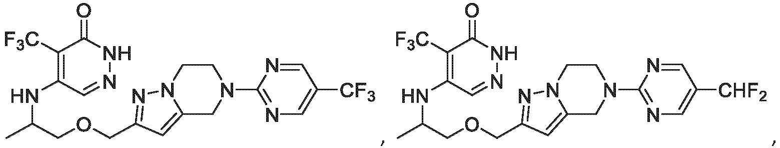 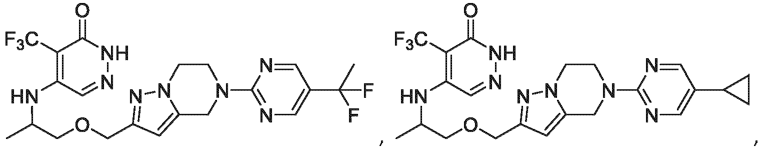 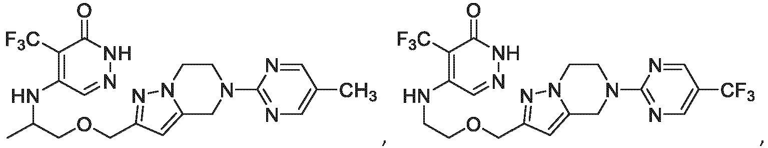 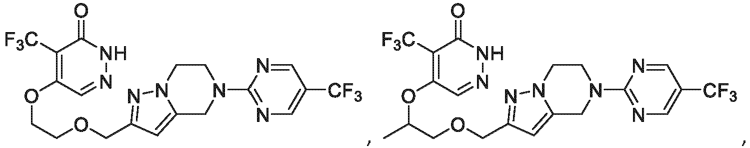 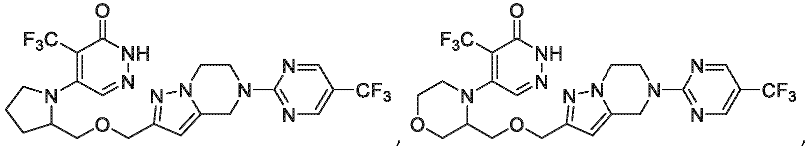 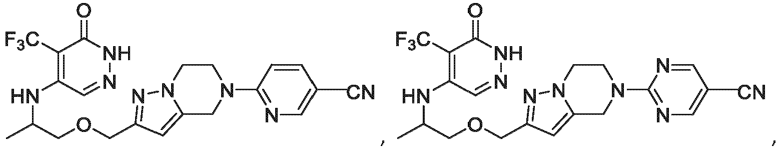 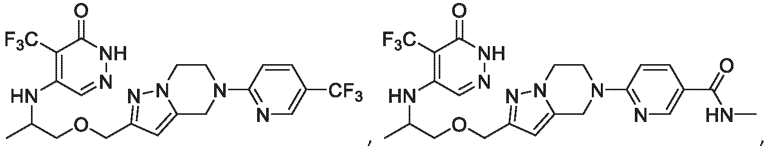 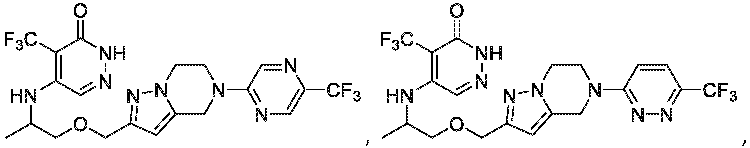 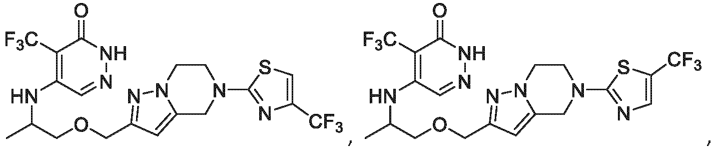 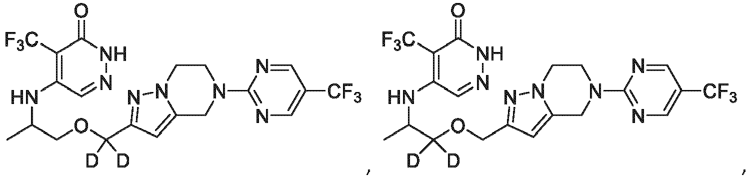 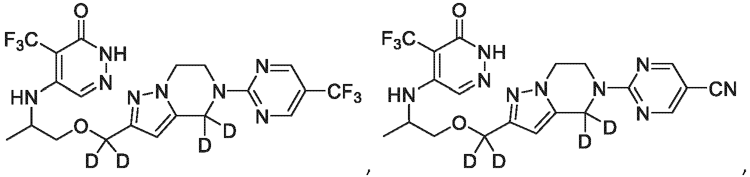 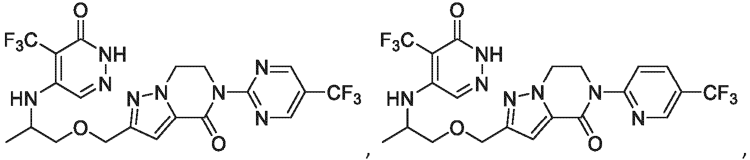 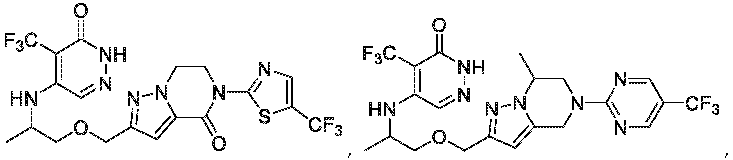 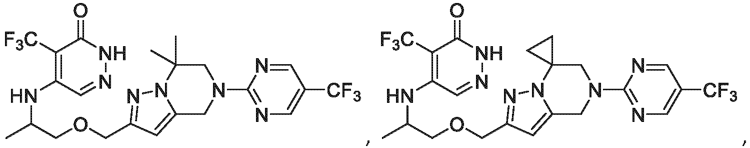 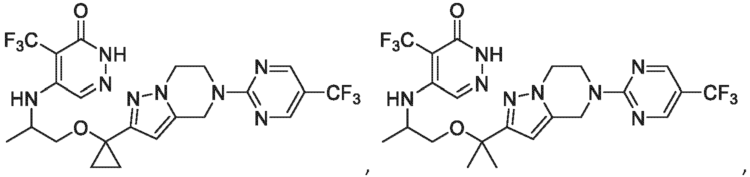 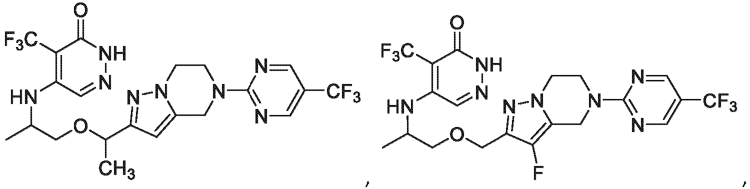 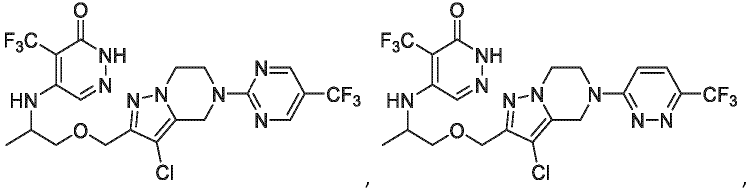 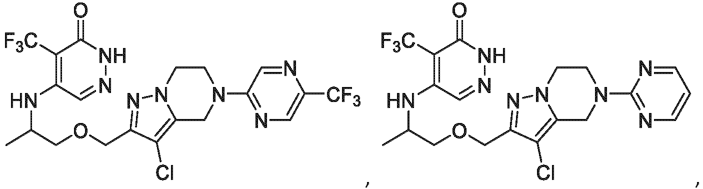 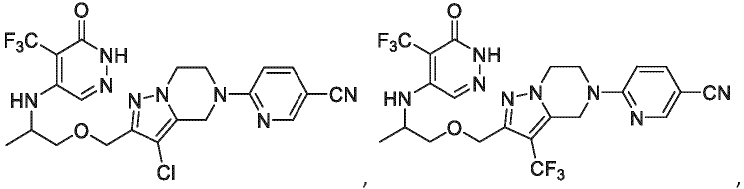 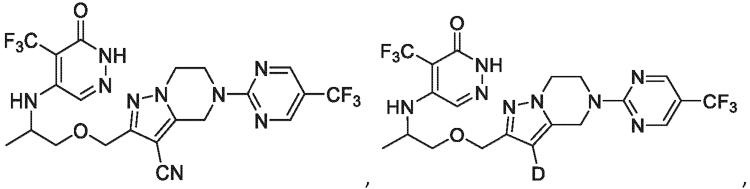 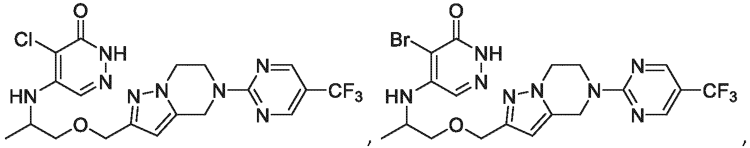 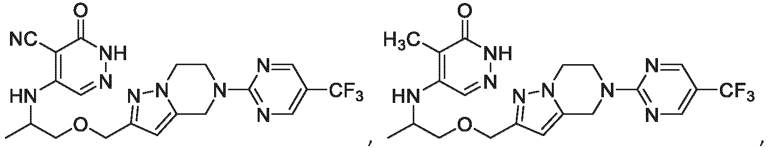 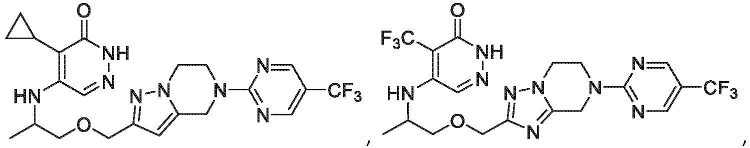 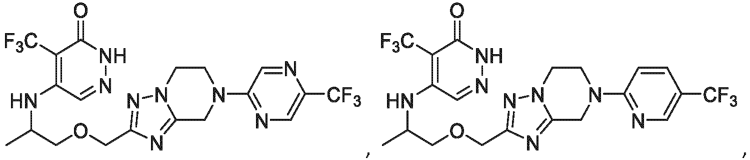 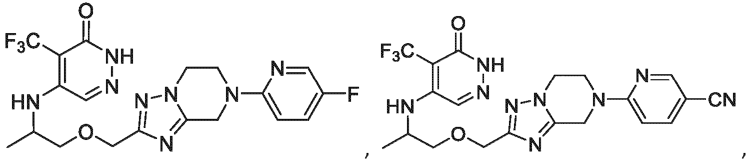 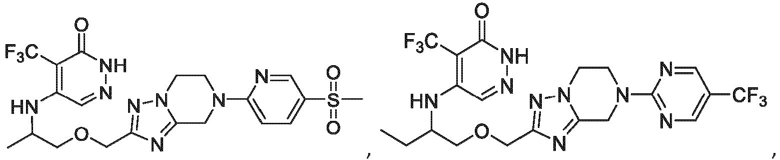 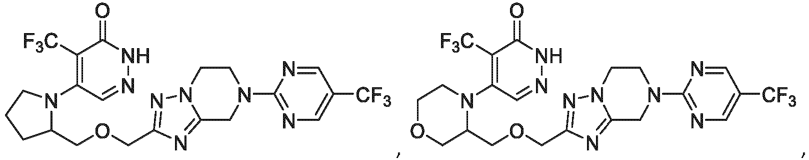 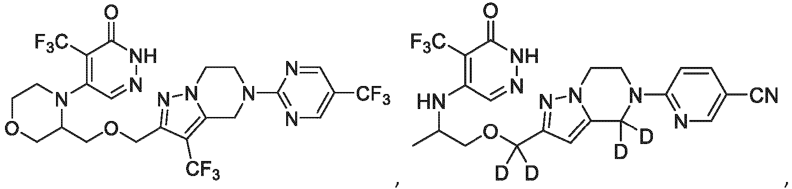 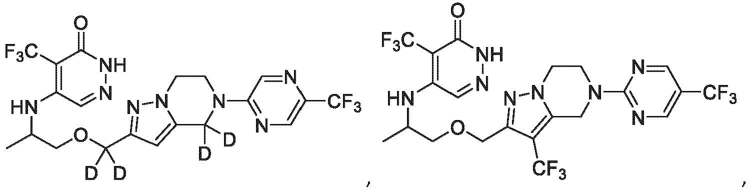 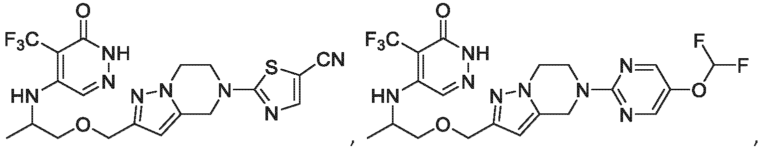 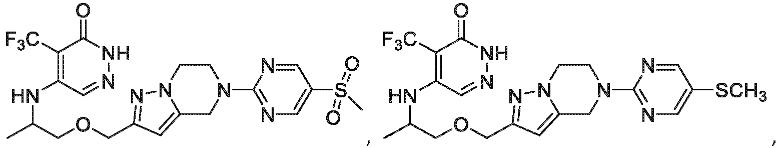 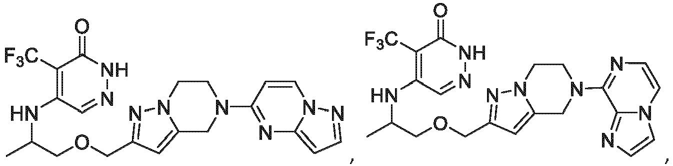 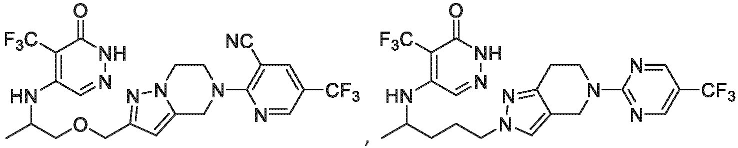 or

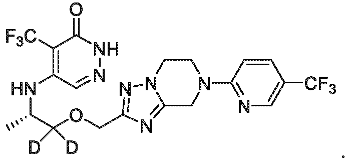

12. The pharmaceutical combination according to claim 1, wherein the component A is Pembrolizumab, Nivolumab, Serplulimab, Pidilizumab, Lambrolizumab, Atezolizumab, Toripalimab, Sintilimab, Tislelizumab, Camrelizumab, Penpulimab, Zimberelimab, Envafolimab, Sugemalimab, Dostarlimab, Cadonilimab, Cemiplimab, Retifanlimab, BMS-986213, HX-008, Geptanolimab, Prolgolimab, Socazolimab, Avelumab, Adebrelimab, or Durvalumab.

13. The pharmaceutical combination of at least two components according to any one of claims 1 to 12, comprising the component A according to claim 12 and the component B according to any one of claims 1 to 11, and one or more pharmaceutically acceptable excipients, diluents or carriers.

14. The pharmaceutical combination according to any one of claims 1 to 13, wherein the total daily dosage of the component B is selected from 50-1500 mg, preferably 100-1000 mg, more preferably 400-800 mg, as measured by the amount of free base.

15. Use of the pharmaceutical combination according to any one of claims 1 to 14 in the treatment or prevention of solid tumors.

16. The use according to claim 15, wherein the component A according to claim 12 and the component B according to any one of claims 1 to 11 are given simultaneously.

17. The use according to claim 15, wherein the component A according to claim 12 and the component B according to any one of claims 1 to 11 are given sequentially.

18. The use according to any one of claims 15 to 17, wherein the component A according to claim 12 is given intravenously at a frequency of twice a week, once a week, once every two weeks, once every three weeks, once every four weeks, once every month, or once every 3-6 months, preferably twice a week, once a week, once every two weeks or once every three weeks.

19. The use according to any one of claims 15 to 17, wherein the component B according to any one of claims 1 to 11 is given orally at a frequency of twice daily or once daily.
